# EUROPÄISCHE PATENTSCHRIFT

(11) **EP 1 824 443 B1**
(45) Veröffentlichungstag und Bekanntmachung des Hinweises auf die Patenterteilung: **14.07.2010**
(21) Anmeldenummer: 05799821.3
(22) Anmeldetag: 28.10.2005
(51) Int. Cl.: A61K 6/06, C04B 35/645

(54) **HYDROXYLAPATIT-WERKSTOFF UND EIN VERFAHREN ZU DESSEN HERSTELLUNG**
HYDROXYLAPATITE MATERIAL AND METHOD FOR THE PRODUCTION THEREOF
MATIERE PREMIERE D'HYDROXYLAPATITE ET PROCEDE POUR LA PRODUIRE

(30) Priorität: 01.11.2004 DE 102004053180; 15.12.2004 DE 102004060745
(43) Veröffentlichungstag der Anmeldung: 29.08.2007
(73) Patentinhaber: Universität Hamburg, 20148 Hamburg (DE); INSTYTUT WYSOKICH CISNIEN POLSKIEJ AKADEMII NAUK, 01-142 Warszawa (PL); Universität Hamburg, 20246 Hamburg (DE)
(72) Erfinder: SHI, Jianmin, M., Sc., 38114 Braunschweig (DE); BISMAYER, Ulrich, 20146 Hamburg (DE); KLOCKE, Arndt, 20149 Hamburg (DE); PALOSZ, Bogdan, PL-03-687 Warszawa (PL); GIERLOTKA, Stanislaw, PL-02-482 Warszawa (PL)
(74) Vertreter: Richter, Joachim
(86) Internationale Anmeldenummer: PCT/EP2005/011578
(87) Internationale Veröffentlichungsnummer: WO 2006/048198

(56) Entgegenhaltungen:
- WO-A-2005/063651
- US-A- 6 013 591
- US-B1- 6 395 214
- SHI, J; BISMAYER, U; KLOCKE, A; GIERLOTKA, S; PALOSZ B: "High-pressure and -temperature sintering of nanosized hydroxyapatite powders" 6TH ASIAN SYMPOSIUM ON BIOMEDICAL MATERIALS, 19. Juli 2004 (2004-07-19), - 22. Juli 2004 (2004-07-22) XP008058155 Chengdu, China & SHI, J; BISMAYER, U; KLOCKE, A; GIERLOTKA, S; PALOSZ B: "High-pressure and -temperature sintering of nanosized hydroxyapatite powders" KEY ENGINEERING MATERIALS, Bd. 288-289, 2005, Seiten 175-178, Switzerland

## Beschreibung

### Anwendungsgebiet

Die Erfindung betrifft einen Hydroxylapatit-Werkstoff und ein Verfahren zu dessen Herstellung.

### Stand der Technik

Eine bedeutende Aufgabe der Zahnheilkunde ist die Behandlung von Zahnkaries. Eine solche Behandlung umfasst die Entfernung der kariös werdenden Zahnhartsubstanz, d. h. des betroffenen Zahnschmelzes und Zahnbeins. Anschließend muss die entfernte Zahnhartsubstanz durch geeignete Materialien ersetzt werden.

Verschiedene Materialien sind für den Ersatz der fehlenden Zahnstruktur verwendet worden. Die Materialien werden dabei in Abhängigkeit von der Methode, wie sie auf die verbliebene Zahnsubstanz aufgebracht werden, unterschieden. Bei der direkten restaurativen Methode wird das verwendete, formbare Material ungeformt, vorzugsweise in einem weichen und anpassungsfähigen Zustand, von einem Zahnarzt in einen Zahnhohlraum eingebracht und erhält erst dort die erwünschte Form. Die für die direkte restaurative Methode geeigneten Materialien werden auch als direkt-restaurative Materialien bezeichnet. Nach einer genauen Anpassung an die erwünschte Form erreichen diese Materialien mechanische Stabilität durch Polymerisation oder Legierungsverfahren. Direkt-restaurative Materialien umfassen Harze (typischerweise bestehend aus einem Kunststoffharzmaterial, einem organischen Haftvermittler und Füllstoffteilchen), Zemente (beispielsweise Zinkphosphatzement, Zinkpolycarboxylatzement, Glasionomerzement, Compomere) und Zahnamalgam (eine Legierung, bestehend aus Ag, Sn, Cu, Hg).

Demgegenüber müssen die bei indirekten restaurativen Methoden verwendeten Materialien in einem Zahnlabor in die erwünschte Form gebracht werden. Die Materialien, die bei einer solchen indirektrestaurativen Methoden eingesetzt werden, werden auch als indirekt-restaurative Materialien bezeichnet. Die indirekten restaurativen Methoden erfordern eine genaue Ansicht der Zahnstruktur, die ersetzt werden soll. Eine solche Ansicht kann beispielsweise durch einen Zahnabdruck der Zähne des Patienten oder mittels eines Scanners erhalten werden. Das Material wird dann im Labor verarbeitet, um die gewünschte Form zu erhalten. Indirekt wiederherstellende Materialien umfassen Legierungen und Dentalkeramiken. Legierungen können auf Gold, Palladium oder Nickel basieren. Um die guten mechanischen Eigenschaften der Metalle mit einer verbesserten Ästhetik, insbesondere der Zahnfarbe, zu kombinieren, wurden Techniken entwickelt, mit denen eine Keramik mit Legierungsmetallen verbunden wurden (erstmals 1959). Vollkeramikmaterialien wurden in den 80iger Jahren eingeführt und ermöglichten eine verbesserte Ästhetik. Dentalkeramiken bestehen hauptsächlich aus SiO₂ und kleineren Mengen an Al₂O₃, MgO und ZrO₂. CAD-CAM-Verfahren sind in den 90iger Jahren eingeführt worden, um aus Keramikblöcken Füllungen (Inlays) und Krone durch Fräsen zu formen.

Allerdings weisen die bisher zur Zahnrestaurierung verwendeten Materialen eine Reihe von Nachteilen auf. Besonders nachteilig ist, dass die Materialien hinsichtlich ihrer chemischen Zusammensetzung der gesunden Zahnsubstanz weder chemisch noch biologisch ähneln. Dies hat Konsequenzen im Hinblick auf die Bioverträglichkeit dieser Materialien, aber auch im Hinblick auf ihr optisches Erscheinungsbild. Beispielsweise sind Zahnamalgame und Metalle nicht zahnfarben. Vollkeramikmaterialien kombinieren hingegen mechanische Stabilität und ästhetische Eigenschaften, so dass sie als indirekt-restaurative Materialien in der Zahlheilkunde geeignet sind. Jedoch unterscheidet sich die Zusammensetzung dieser Keramiken stark von denen der Zahnhartsubstanz. Deshalb zeigen diese Materialien Eigenschaften, insbesondere Härte und Brüchigkeit die sich von den Eigenschaften der natürlichen Zahnsubstanz deutlich unterscheidet. Sie weisen daher nur eine eingeschränkte Bioverträglichkeit auf.

Hydroxylapatit-Werkstoffe der eingangs genannten Art offenbart auch das Dokument SHI, J; BISMAYER, U; KLOCKE, A; GIERLOTKA, S; PALOSZ B: "High-pressure and -temperature sintering of nanosized hydroxylapatite powders" 6TH ASIAN SYMPOSIUM ON BIOMEDICAL MATERIALS 19. Juli 2004 - 22. Juli 2004 Chengdu, China und das US-A-6 013 591. Diese bekannten Hydroxylapatit-Werkstoffe weisen jedoch nur eine begrenzte Stabilität auf.

### Aufgabe, Lösung, Vorteil

Aufgabe der Erfindung ist es, die Nachteile nach dem Stand der Technik zu beseitigen. Es soll insbesondere ein Werkstoff angegeben werden, der über Eigenschaften verfügt, die den Eigenschaften des anorganischen Anteils von Zahnschmelz weitgehend gleichen. Insbesondere soll der Hydroxylapatit-Werkstoff eine hohe mechanische Festigkeit sowie geringe Brüchigkeit aufweisen und über eine hohe Biokompatibilität verfügen. Ferner sollen ein Verfahren zu Herstellung des Hydroxylapatit-Werkstoffes sowie eine Verwendung derselben angegeben werden.

Diese Aufgabe wird durch die Merkmale der Ansprüche 1 und 6 gelöst. Zweckmäßige Ausgestaltungen der Erfindungen sind Gegenstand der Unteransprüche.

Nach Maßgabe der Erfindung ist ein Hydroxylapatit-Werkstaff vorgesehen, der durch
(a) Bereitstellen von nanokristallinem Hydroxylapatit, das eine Partikelgröße unter 100 nm, bevorzugterweise von 30 nm bis 100 nm aufweist;
(b) Vorpressen bei Raumtemperatur des in (a) bereitgestellten nanokristallinen Hydroxylapatit bei 10 MPa; und
(c) Behandeln des nanokristallinen Hydroxylapatits bei einem Druck von 1,4 bis 7,7 GPa und einer Temperatur von 200 bis 700° C.
erhalten wird.

Die Erfindung basiert auf der Tatsache, dass die erkrankte Zahnstruktur bei der konservierenden Stomatologie vorzugsweise durch Materialien ersetzt werden sollte, die der natürlichen Zahnsubstanz weitgehend gleichen. Zahnschmelz besteht zu ca. 95 % aus anorganischen Verbindungen, wobei der Anteil von Hydroxylapatit an diesen Verbindungen 90 % beträgt. Die Hauptkomponente von humanen Zähnen ist Hydroxylapatit. Erfindungsgemäß liegt also ein biomimetischer Werkstoff, der der Restauration von Zähnen dient, vor, wobei der biomimetische Werkstoff ein bei einem Druck von 1,4 bis 7,7 GPA und einer Temperatur von 200 bis 900° C ausgebildeter nanokristalliner Hydroxylapatit ist.

Es war bisher jedoch nicht möglich, einen Werkstoff, der ausschließlich oder überwiegend aus Hydroxylapatit bestand, zur Restauration von Zähen zu verwenden. Ursache hierfür war insbesondere, dass der synthetisch hergestellte Hydroxylapatit nicht über die notwendigen Eigenschaften verfügt. Insbesondere wies dieser Hydroxylapatit nicht die notwendige hohe Härte und geringe Brüchigkeit auf. Außerdem zersetzte sich der Hydroxylapatit, wenn er höheren Temperaturen ausgesetzt wurde, zu anderen Calciumphosphaten.

Überraschenderweise zeigte sich, dass der mittels des erfindungsgemäßen Verfahrens hergestellte Hydroxylapatit-Werkstoff über eine hohe Mikrohärte und eine geringe Brüchigkeit verfügt. Dies wurde erreicht, indem er hohen Drücken und Temperaturen ausgesetzt wurde und so verdichtet wurde. Überdies verfügt der erfindungsgemäße Werkstoff über ein optisches Erscheinungsbild, das dem im Zahnschmelz und Zahnbein vorkommenden, natürlichen Hydroxylapatit insbesondere im Hinblick auf Farbe und Transluzenz gleicht.

Der erfindungsgemäße Hydroxylapatit-Werkstoff ähnelt somit in seinen Eigenschaften, insbesondere seinen mechanischen und ästhetischen Eigenschaften dem anorganischen Anteil von natürlichem Zahnschmelz und Zahnbein weitgehend. Der erfindungsgemäße Hydroxylapatit-Werkstoff ist somit ein biomimetischer Werkstoff.

Der erfindungsgemäße Hydroxylapatit-Werkstoff kann organische oder anorganische Additive zur mechanischen und chemischen Stabilisierung enthalten, die dem Hydroxylapatit vor der Anwendung von Druck und Temperatur zugemischt werden.

Zur Herstellung des erfindungsgemäßen Hydroxylapatit-Werkstoffes wird ein nanokrostalliner Hydroxylapatit verwendet. Unter dem Begriff "nanokristallin" wird ein Hydroxylapatit der Partikelgröße 30 nm bis 100 nm, gefällt als Präzipitat aus wässriger Lösung verstanden.

Als Ausgangsstoff kann jedes synthetische oder natürliche Hydroxylapatit verwendet werden, das nanokrostallin ist. Vorzugsweise wird ein Hydroxylapatitpulver als Ausgangsstoff verwendet, wobei die Partikelgröße des Hydroxylapatitpulvers im Nanometerbereich liegt, vorzugsweise 30 nm bis 100 nm beträgt.

Nach dem Bereitstellen des nanokristallinen Hydroxylapatits wird dieses bei einem Druck von 1,4 bis 7,7 GPa und einer Temperatur von 200 bis 900° C behandelt. Bevorzugt wird eine Behandlung des nanokristallinen Hydroxylapatits bei einem Druck von 1,4 bis 7,7 GPa und einer Temperatur von 200 bis 700° C; besonders bevorzugt bei einem Druck von 2,0 bis 2,5 GPa und einer Temperatur von 200 bis 550° C durchgeführt.

Mittels des erfindungsgemäßen Verfahrens wird ein fester Hydroxylapatit-Block erhalten, der als indirekt-restauratives Material verwendet werden kann. Der Hydroxylapatit-Block kann unter Verwendung bekannter Maßnahmen wie beispielsweise CAD/CAM-Verfahren in Zahnlabors oder Zahnarztpraxen zur Herstellung von Zahnkronen, Inlays, Outlays verwendet werden. Dazu wird aus dem Hydroxylapatit-Block die vorgegebene Form beispielsweise mittels einer Fräsmaschine ausgefräst.

Der erfindungsgemäße Hydroxylapatit-Werkstoff ist als restauratives Material in der konservierenden Stomatologie sehr gut geeignet und kann somit zur Füllungs-, Kronen- und Brückentherapie verwendet werden. Er ist als Ersatz (z. B. Kronen- und Wurzelersatz) für Zahnhartsubstanzen und deren Reparatur aufgrund seiner Biokompatibilität, mechanischen Eigenschaften und optischem Erscheinungsbild ausgezeichnet geeignet.

### Kurzbeschreibung der Zeichnung

Die Erfindung wird anschließend anhand von Zeichnungen näher erläutert. Dabei zeigen:
- Fig. 1: eine als Hochdruck-, Hochtemperatur-Zelle ausgebildete Vorrichtung zur Ausführung des erfindungsgemäßen Verfahrens;
- Fig. 2: photographische Aufnahmen von Beispielen des erfindungsgemäßen Hydroxylapatit-Werkstoffes;
- Fig. 3a, b: rasterelektronenmikroskopische Aufnahmen von Beispielen des erfindungsgemäßen Hydroxylapatit-Werkstoffes;
- Fig.4: Röntgenbeugungsdiagramme von Beispielen des erfindungsgemäßen Hydroxylapatit-Werkstoffes sowie
- Fig. 5: Infrarotabsorptionsspektren von Beispielen des erfindungsgemäßen Hydroxylapatit-Werkstoffes.

### Detaillierte Beschreibung der Erfindung und bester Weg zur Ausführung der Erfindung

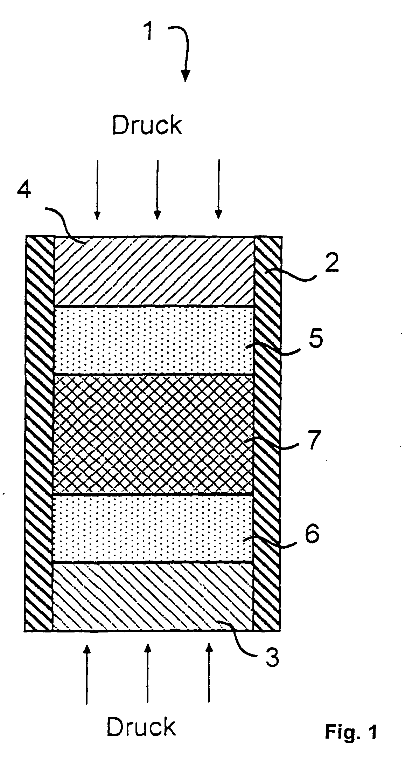

Die in Fig. 1 als Hochdruck-, Hochtemperatur-Zelle gezeigte Vorrichtung 1 wurde verwendet, um die erfindungsgemäßen Hydroxylapatit-Werkstoffe herzustellen. Die Vorrichtung ist eine Hochdruck/Hochtemperatur-Zelle. Diese Vorrichtung 1 besteht aus zwei gegenüberliegenden Stempeln 3, 4 und Graphit-Heizungen 5, 6 in einem zylinderförmigen temperatur- und druckfesten Gehäuse 2. Das Hydroxylapatit-Pulver wird in die Vorrichtung 1 zwischen die Stempel 3, 4 und die Graphit-Heizungen 5, 6 eingebracht. Über die Stempel 3, 4 wird auf das Hydroxylapatit-Pulver 7 der vorgegebene Druck ausgeübt (siehe Pfeile). Die zum erreichen der notwendigen Temperaturen erforderliche Wärmemenge wird über die Graphit-Heizungen 5, 6 in das Hydroxylapatit-Pulver 7 eingebracht. Nach der Behandlung wird der erfindungsgemäße Hydroxylapatit-Werkstoff in Form eines Hydroxylapatit-Zylinders aus der Vorrichtung 1 entnommen.

### Beispiele 1 bis 5

100 mg farbloser Hydroxylapatit (Berkeley Advanced Biomaterials INC, USA) mit einer durchschnittlichen Partikelgröße unter 100 nm wurden in eine Hochdruck-, Hochtemperatur-Zelle (Fig. 1) eingebracht. Die Probe wird zunächst bei Raumtemperatur bei 10 MPa vorgepresst, dann auf die in Tabelle 1 angegebene Temperatur erwärmt und dabei dem gleichfalls in Tabelle 1 angegebenen Druck für einen Zeitraum von einer Minute ausgesetzt. Die Erwärmungsgeschwindigkeit betrug 200° C/h. Die Probe wurde bei der angegebenen Temperatur und dem angegebenen Druck gehalten und anschließend mit einer Abkühlungsgeschwindigkeit von 200° C/h abgekühlt. Der so erhaltene Hydroxylapatit wurde nach dem Abkühlen auf Raumtemperatur aus der Hochdruck/Hochtemperatur-Zelle entnommen. Anschließend wurden die Proben folgenden Tests unterzogen:

**Tabelle 1. Mechanische Eigenschaften der erfindungsgemäßen Materialien**

| Probe | Temperatur (°C) | Druck (GPa) | Mikrohärte (GPa) | Bruchzähigkeit (MPa·m^{1/2}) |
|---|---|---|---|---|
| 1 | 200 | 2,5 | 4,95 ± 0,71 | 0,60 ± 0,14 |
| 2 | 200 | 7,7 | 3,58 ± 0,21 | 0,59 ± 0,08 |
| 3 | 350 | 2,5 | 5.28 ± 0,70 | 0,50 ± 0,13 |
| 4 | 500 | 2,5 | 5,38 ± 0,64 | 0,60 ± 0,15 |
| 5 | 700 | 2,5 | 5,26 ± 0,36 | 1,00 ± 0,20 |

### Bestimmung der Mikrohärte

Die Bestimmung der Mikrohärte erfolgte gemäß Standard nach Knoop (ASTM E384 und Vickers (DHP). Dazu wurde in die jeweilige Hydroxylapatit-Probe ein Indenter jeweils 10-15 Sekunden eingedrückt. Die Mikrohärte wurde nach Standardverfahren aus dem Eindruck ermittelt.

Die Ergebnisse der Mikrohärte-Bestimmung sind in Tabelle 1 angegeben. Alle Hydroxylapatit-Proben zeigten eine hervorragende Mikrohärte, die der von natürlichem Zahnschmelz glich.

### Bestimmung der Bruchzähigkeit

Die Bruchzähigkeit wurde ebenfalls aus den Vickers-Eindruckfiguren ermittelt (Literatur: Anstis et al. 1981, American Ceramic Society Vol 64, S. 533 ff.).

Die Ergebnisse der Bruchzähigkeits-Bestimmung sind in Tabelle 1 angegeben. Alle Hydroxylapatit-Proben zeigten eine hervorragende Bruchzähigkeit, die der von natürlichem Zahnschmelz glich.

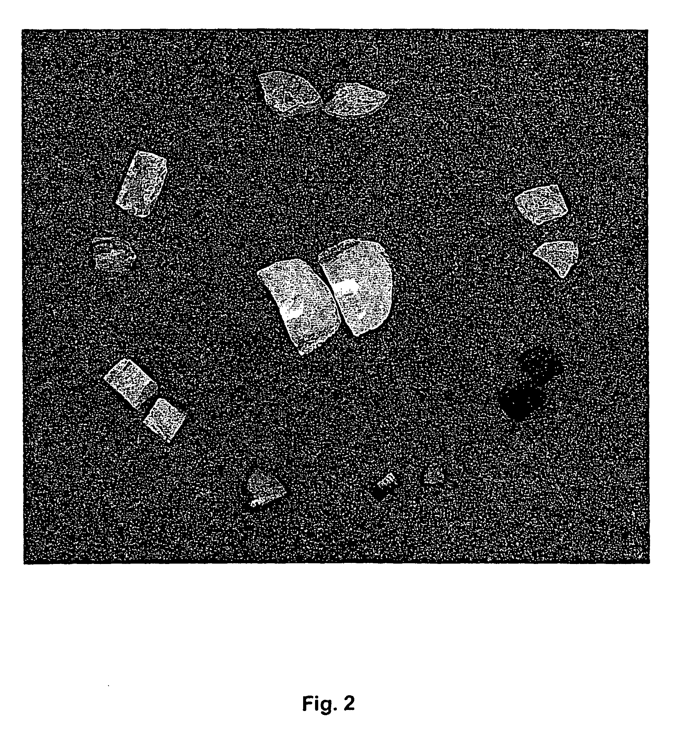

Fig. 2 ist eine photographische Aufnahme, die die Hydroxylaptit-Proben der Beispiele 1 bis 5 zeigt. Zu erkennen ist, dass das optische Erscheinungsbild der Proben aus dem erfindungsgemäßen Werkstoff hinsichtlich ihrer Transluzenz und Farben natürlichem Zahnschmelz gleicht. Die Partikelgröße ist kleiner 100 nm. Die Werkstoffe sind gekennzeichnet durch Druck/Temperaturbedingungen. Die beiden Materialien unter den Bedingungen Raumtemperatur (RT)/7,7 GPa und 200° C/5 GPa sind nicht in der Tabelle 1 enthalten. Im Zentrum der Abbildung befindet sich eine natürliche Probe humanen Zahnschmelzes.

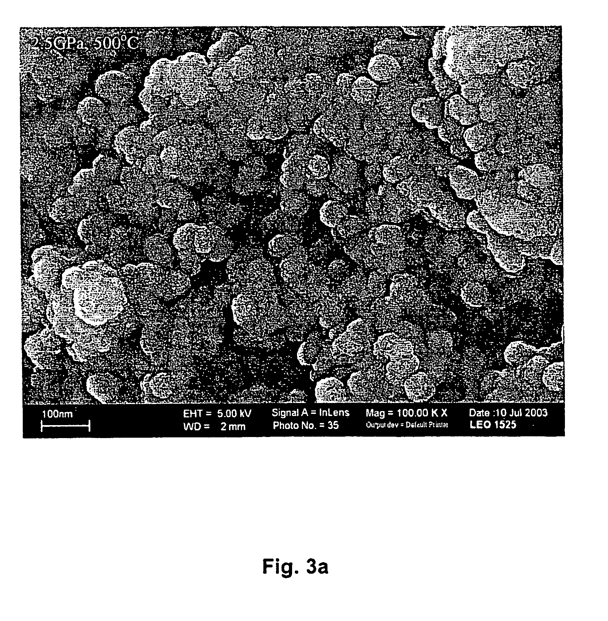

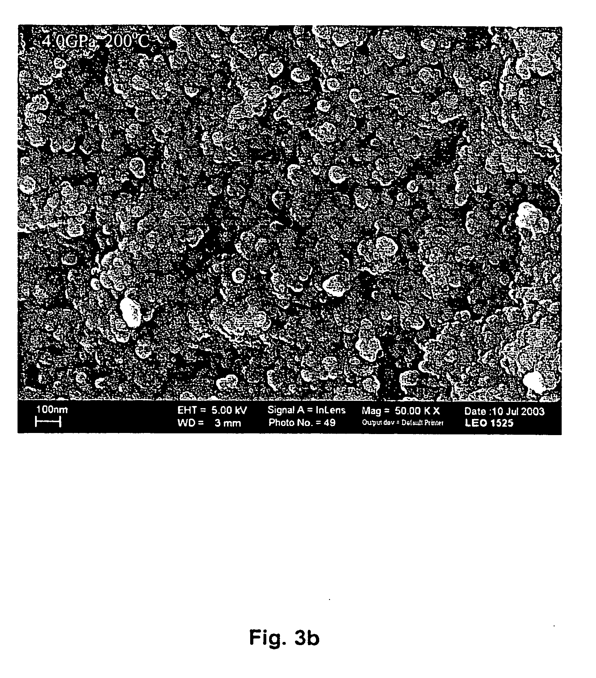

Fig. 3a und 3b zeigen rasterelektronenmikroskopische Aufnahmen von beispielhaften Hydroxylapatit-Proben. Fig. 3a zeigt eine Aufnahme einer Probe gemäß Beispiel 4 von Tabelle 1, wobei die Temperatur 500° C und der Druck 2,5 GPa betrugen. Fig. 3b zeigt eine Aufnahme einer Probe, die ebenso wie die in den Beispielen 1 bis 5 hergestellt worden ist, wobei die Temperatur 200° C und der Druck 4,0 GPa betrugen. In diesen Aufnahmen ist deutlich die dreidimensionale Netzwerkstruktur des erfindungsgemäßen Werkstoffes zu erkennen, die eine Verbesserung der Härte und Bruchfestigkeit des Hydroxylapatit-Werkstoffes gegenüber den bisher bekannten Materialien bewirkt.

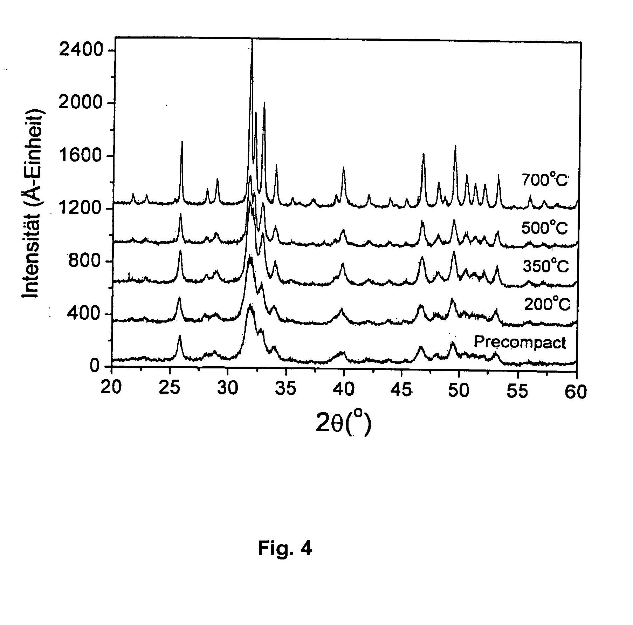

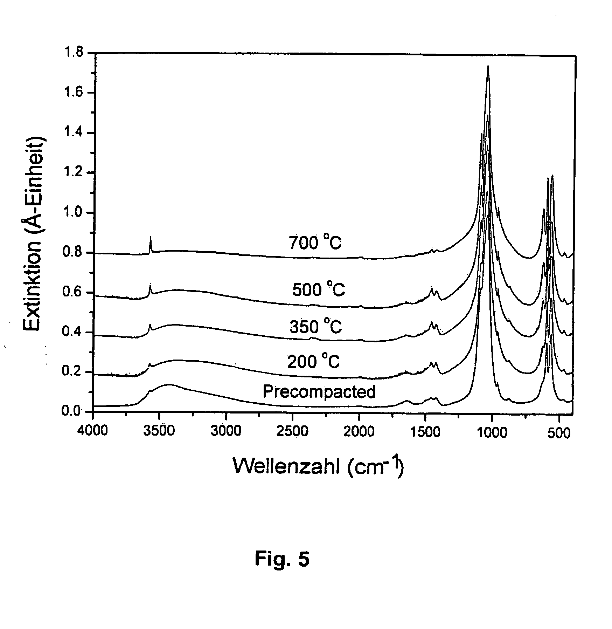

Fig.4 zeigt ein Pulver-Röntgenbeugungsdiagramm von beispielhaften Proben, die ebenso wie die Beispiele 1 bis 5 hergestellt wurden, außer dass bei den Proben ein Druck von 2,5 GPa bei den in Fig. 4 gezeigten Temperaturen angewendet wurde. Fig. 5 zeigt Infrarotabsorptionsspektren der in Fig.4 gezeigten Proben. Die mit "Precompact" bezeichneten Graphen in beiden Figuren zeigen eine unbehandelte Hydroxylapatit-Probe, wie sie bei der Herstellung der erfindungsgemäßen Hydroxylapatit-Werkstoffe verwendet wurde. Diese unbehandelte Probe entspricht dem in den Beispielen 1 bis 5 eingesetzten Ausgangsstoff.

Die Figuren 5 und 4 zeigen, dass bei Anwendungen des erfindungsgemäßen Verfahrens keine Zersetzung des Hydroxylapatits zu anderen Calciumphosphat-Phasen erfolgt.

Als Additive, die in kleinen Mengen dem nano-Hydroxylapatit zugesetzt werden, kann u. a. bevorzugterweise als Stabilisator "Seide" als organische Komponente eingesetzt werden.

### Bezugszeichenliste

- 1: Hochdruck/Hochtemperatur-Zelle
- 2: Gehäuse
- 3, 4: Bornitrid-Stempel
- 5,6 ,: Graphit-Heizungen
- 7: Hydroxylapatit

## Patentansprüche

1. Hydroxylapatit-Werkstoff, erhältlich durch
(a) Bereitstellen von nanokristallinem Hydroxylapatit, das eine Partikelgröße unter 100 nm, bevorzugterweise von 30 nm bis 100 nm aufweist;
(b) Vorpressen bei Raumtemperatur des in (a) bereitgestellten nanokristallinen Hydroxylapatit bei 10 MPa; und
(c) Behandeln des nanokristallinen Hydroxylapatits bei einem Druck von 1,4 bis 7,7 GPa und einer Temperatur von 200 bis 700° C.

2. Hydroxylapatit-Werkstoff nach Anspruch 1,
**dadurch gekennzeichnet,**
**dass** organische oder anorganische Additive zur mechanischen und chemischen Stabilisierung integriert sind.

3. Hydroxylapatit-Werkstoff nach Anspruch 1 oder2,
**dadurch gekennzeichnet,**
**dass** das Behandeln des nanokristallinen Hydroxylapatits bei einem Druck von 2,0 bis 2,5 GPa und einer Temperatur von 200 bis 550° C durchgeführt wird.

4. Verfahren zur Herstellung eines Hydroxylapatit-Werkstoffes, umfassend
(a) Bereitstellen von nanokristallinem Hydroxylapatit das eine Partikelgröße unter 100 nm, bevorzugterweise von 30 nm bis 100 nm aufweist;
(b) Vorpressen des in (a) bereitgestellten nanokristallinen Hydroxylapatit bei 10 MPa; und
(c) Behandeln des nanokristallinen Hydroxylapatits bei einem Druck von 1,4 bis 7,7 GPa und einer Temperatur von 200 bis 700 °C.

5. Verfahren nach Anspruch 4,
**dadurch gekennzeichnet,**
**dass** das Behandeln des nanokristallinen Hydroxylapatits bei einem Druck von 2,0 bis 2,5 GPa und einer Temperatur von 200 bis 550° C durchgeführt wird.

6. Verwendung eines Hydroxylapatit-Werkstoffes nach einem der Ansprüche 1 bis 3 bei der Behandlung von dentalen Erkrankungen.

7. Verwendung eines Hydroxylapatit-Werkstoffes nach einem der Ansprüche 1 bis 3 zur Herstellung eines Zahn-Inlays.

8. Verwendung eines Hydroxylapatit-Werkstoffes nach einem der Ansprüche 1 bis 3 zur Herstellung einer Zahnkrone.

## Claims

1. A hydroxylapatite material obtained by
(a) providing nanocrystalline hydroxylapatite having a particle size of less than 100 nm, preferably 30 nm to 100 nm;
(b) prepressing the nanocrystalline hydroxylapatite provided in (a) at 10 MPa at room temperature; and
(c) treating said nanocrystalline hydroxylapatite at a pressure of 1.4 to 7.7 GPa and a temperature of 200 to 700 °C.

2. The hydroxylapatite material according to claim 1,
**characterized in that**
organic or inorganic additives are integrated for mechanical and chemical stabilization.

3. The hydroxylapatite material according to claim 1 or claim 2,
**characterized in that**
treating said nanocrystalline hydroxylapatite is performed at a pressure of 2.0 to 2.5 GPa and a temperature of 200 to 550 °C.

4. A method for producing a hydroxylapatite material comprising
(a) providing nanocrystalline hydroxylapatite having a particle size of less than 100 nm, preferably 30 nm to 100 nm;
(b) prepressing the nanocrystalline hydroxylapatite provided in (a) at 10 MPa; and
(c) treating said nanocrystalline hydroxylapatite at a pressure of 1.4 to 7.7 GPa and a temperature of 200 to 700 °C.

5. The method according to claim 4,
**characterized in that**
treating said nanocrystalline hydroxylapatite is performed at a pressure of 2.0 to 2.5 GPa and a temperature of 200 to 550 °C.

6. The use of a hydroxylapatite material according to any of claims 1 to 3 in the treatment of dental diseases.

7. The use of a hydroxylapatite material according to any of claims 1 to 3 for producing a dental inlay.

8. The use of a hydroxylapatite material according to any of claims 1 to 3 for producing a dental crown.

## Revendications

1. Matériau hydroxyapatite, pouvant être obtenue par
(a) la mise à disposition d'une hydroxyapatite nanocristalline dont la taille des particules est inférieure à 100 nm, de préférence comprise entre 30 nm et 100 nm;
(b) le pré-pressage à 10 MPa à température ambiante de l'hydroxyapatite nanocristalline mise à disposition dans l'étape (a); et
(c) le traitement de ladite hydroxyapatite nanocristalline à une pression comprise entre 1,4 et 7,7 GPa et une température comprise entre 200 et 700 °C.

2. Matériau hydroxyapatite selon la revendication 1,
**caractérisé en ce**
**que** des additifs organiques ou inorganiques destinés à la stabilisation mécanique et chimique y sont intégrés.

3. Matériau hydroxyapatite selon les revendications 1 ou 2,
**caractérisé en ce**
ledit traitement de ladite hydroxyapatite nanocristalline est réalisé à une pression comprise entre 2,0 et 2,5 GPa et une température comprise entre 200 et 550 °C.

4. Procédé de préparation d'un matériau hydroxyapatite, comprenant
(a) la mise en oeuvre d'une hydroxyapatite nanocristalline dont la taille des particules est inférieure à 100 nm, de préférence comprise entre 30 nm et 100 nm;
(b) le pré-pressage à 10 MPa de l'hydroxyapatite nanocristalline mise à disposition dans l'étape (a); et
(c) le traitement de ladite hydroxyapatite nanocristalline à une pression comprise entre 1,4 et 7,7 GPa et une température comprise entre 200 et 700 °C.

5. Procédé selon la revendication 4,
**caractérisé en ce**
ledit traitement de ladite hydroxyapatite nanocristalline est réalisé à une pression comprise entre 2,0 et 2,5 GPa et une température comprise entre 200 et 550 °C.

6. Utilisation d'un matériau hydroxyapatite selon l'une des revendications 1 à 3 dans le traitement d'affections dentaires.

7. Utilisation d'un matériau hydroxyapatite selon l'une des revendications 1 à 3 pour fabriquer un inlay dentaire.

8. Utilisation d'un matériau hydroxyapatite selon l'une des revendications 1 à 3 pour fabriquer une couronne dentaire.
